# EUROPEAN PATENT APPLICATION

(11) **EP 2 151 451 A1**
(43) Date of publication of application: **10.02.2010**
(21) Application number: 08722517.3
(22) Date of filing: 19.03.2008
(51) Int. Cl.: C07K 5/072, A23L 1/305, A61K 38/00, A61P 1/16, A61P 31/12, C07K 5/113

(54) **COMPOSITION FOR PREVENTING OR TREATING LUNG DISEASE**

(30) Priority: 23.03.2007 JP 2007077594
(71) Applicant: Nisshin Pharma Inc., Tokyo 101-8441 (JP); National University Corporation Chiba University, Chiba-shi Chiba, 2638522 (JP)
(72) Inventor: SANADA, Hiroo, Matsudo-shi Chiba 271-8510 (JP); SATO, Kenji, Kyoto-shi Kyoto 606-8522 (JP); ONO, Shin, Toyama-shi Toyama 930-8555 (JP); SUZUKI, Yoshio, Tokyo 101-8441 (JP)
(74) Representative: Schrell, Andreas
(86) International application number: PCT/JP2008/055143
(87) International publication number: WO 2008/117730

(57) **Abstract**

This invention provides a composition having preventive and/or therapeutic effects on liver disease, which is highly effective, free of side effects, easily ingestible, and capable of long-term ingestion from the viewpoint of a cost and safety. This invention relates to a peptide having the amino acid sequence represented by the following formula: pyroGlu-(X)n-A, wherein X is the same or different and represents Gln or Asn; A represents Gln, Asn, Leu, Ile, or Val; and n is an integer from 0 to 2 or a salt thereof.

## Description

### Technical Field

The present invention relates to a peptide having preventive and/or therapeutic activity for a liver disease and a preventive and/or therapeutic composition for a liver disease comprising the peptide.

### Background Art

Liver diseases, such as acute hepatitis, chronic hepatitis, alcoholic liver injury, and fatty liver, are socially serious issues of concern. When symptoms worsen and cirrhosis develops, in particular, esophageal varices, hepatic encephalopathy, or the like is induced, and the prognosis for a patient with such disease would be very poor. Further, kidney disease or diabetes may develop as a complication, and development of preventive or therapeutic methods for the same has been awaited. These liver diseases have been found to be induced by various causes. Examples thereof include hepatitis A, hepatitis B, hepatitis C, hepatitis D, hepatitis E, hepatitis F, hepatitis G, and hepatitis TT induced by hepatitis virus infection; alcoholic fatty liver or cirrhosis caused by excessive consumption of alcohol; drug-induced hepatitis caused by drug intake; and autoimmune hepatitis caused by immunopathy.

Pharmaceutical products, such as glycyrrhizin preparations, lamivudine, polyenephosphatidylcholine, malotilate, diisopropylamine dichloroacetate, tiopronin, protoporphyrin sodium, methylmethionine sulfonium chloride, interferon, ursodeoxycholic acid, *Sho-saiko-to,* liver hydrolysate, and glutathione, are used for treatment of such liver diseases. The effects of these drugs to rapidly ameliorate liver diseases, however, are weak and treatment with the use of such drugs is often time-consuming. Even when a liver disease can be rapidly ameliorated to some extent, the GOT, GPT, and γ-GTP levels after amelioration are often as high as the upper limit of the normal levels. When drugs have therapeutic effects on liver diseases, further, adverse reactions disadvantageously occur.

Since the liver metabolizes nutrients such as proteins in the body, treatment of liver diseases has been attempted with the use of amino acids or peptides. For example, JP Patent Publication (kokai) No. H11-171763 A (1999) discloses a therapeutic composition for a liver disease comprising, as an active ingredient, the amino acid valine and comprising no amino acid except for valine as an active ingredient. However, amino acids have a distinctive strong flavor, and it is thus difficult to continuously ingest them.

WO 2002-072131 discloses a drug for a liver disease comprising, as an active ingredient, a peptide comprising four amino acids; i.e., leucine, serine, lysine, and leucine. However, the effects of such peptide have been confirmed only via intraperitoneal administration, and it is unknown whether or not such peptide exhibits its effects via oral administration.

Thus, development of a preventive or therapeutic drug and food product for a liver disease, which has a good flavor, is easily ingestible on a routine basis, exhibits excellent therapeutic effects on a liver disease via oral administration, and is highly safe, has been awaited.

### Disclosure of the Invention

An object of the present invention is to provide a composition having preventive and/or therapeutic effects on a liver disease, which is highly effective, free of side effects, easily ingestible, and capable of long-term ingestion from the viewpoint of a cost and safety.

The present inventors have conducted concentrated studies in order to search for a substance having satisfactory effects of ameliorating liver diseases. As a result, they discovered that administration of a peptide with a certain sequence would improve the GOT level and the GPT level of a patient with a liver disease within a short period of time to result in a normal level or a value close thereto, and the liver disease could then be ameliorated.

Specifically, the present invention includes the following inventions.
(1) A peptide consisting of the amino acid sequence represented by the following formula:

   pyroGlu-(X)n-A

   wherein X is the same or different and represents Gln or Asn; A represents Gln, Asn, Leu, Ile, or Val; and n is an integer from 0 to 2, or a salt thereof.
(2) The peptide or salt thereof according to (1), wherein X represents Gln; A represents Gln, Leu, or Ile; and n is 0 or 1.
(3) A preventive and/or therapeutic composition for a liver disease comprising, as an active ingredient, at least one of the peptides or salts thereof according to (1) or (2).
(4) The composition according to (3), wherein the liver disease is viral hepatitis, an alcoholic liver disease, or a drug-induced liver disease.
(5) The composition according to (3) or (4), which is in the form of a food product.

The present invention can provide a peptide having preventive and/or therapeutic activity for a liver disease, which is superior to a therapy using conventional pharmaceutical products in terms of its high safety and easy ingestion, and a preventive and/or therapeutic composition for a liver disease.

This description includes part or all of the contents as disclosed in the claims and the description of Japanese Patent Application No. 2007-77594, which is a priority document of the present application.

### Best Modes for Carrying out the Invention

Hereafter, preferable embodiments of the present invention are described in detail. In the present invention, the term "liver disease" refers to a symptom of hepatic insufficiency. Examples of liver diseases include inflammatory liver diseases, such as viral hepatitis, alcoholic hepatitis, and drug-induced hepatitis, fatty liver, and cirrhosis. Even when a disease name is unidentified, symptoms of abnormality in indicators for liver functions, such as GOT, GPT, and γ-GTP, are within the scope of liver diseases. Preventive and/or therapeutic activity for a liver disease can be evaluated by assaying liver functions; i.e., by assaying the indicators for liver functions as described above. For example, such activity can be evaluated by assaying the levels of GOT and GPT that are released and concentrated in the blood upon destruction of hepatocytes; i.e., the blood serum markers for liver diseases. More specifically, an active ingredient may be administered to an animal that has developed a liver disease by D-galactosamine and the activity of suppressing the liver disease may then be assayed.

Glutamic oxaloacetic transaminase (GOT) is present in cells of organs or tissues, such as cardiac muscle, the liver, skeletal muscle, and the kidney in large quantities, and GOT is an enzyme that transforms an amino group to form an amino acid. Because GOT is an enzyme that constantly generates and disintegrates in cells that constitute the human body under normal conditions, a given amount of GOT is always contained in the blood of a healthy individual. When an abnormality develops in the above-described organs, however, large quantities of GOT are released into the blood. Thus, GOT content in the blood (i.e., the GOT level) is considered to be an important indicator when diagnosing the occurrence of a disease in organs or body tissue. When a person is afflicted with a liver disease and hepatocytes are destroyed, GOT is released in amounts exceeding the normal level. Thus, assay thereof enables deduction of the type, severity, and the like of a liver disease. A normal GOT level in a human is generally 11 to 40 IU/1.

Glutamic pyruvic transaminase (GPT) is an enzyme that is involved in amino acid formation, as is GOT, and it is present specifically in hepatocytes in large quantities. GPT is present in the kidney in an amount that is about one-third of the amount thereof in the liver, and the presence thereof in other organs is small. A given amount of GPT is always contained in the blood of a healthy individual. Since GPT content in the blood (i.e., the GPT level) increases in response to hepatic disorders such as denaturation or necrosis of hepatocytes, it is considered to be an important indicator when diagnosing liver diseases. When a person is afflicted with a liver disease and hepatocytes are destroyed, GPT is released in amounts exceeding the normal level. Thus, assay thereof enables deduction of the type, severity, and the like of a liver disease. A normal GPT level in a human is generally 6 to 43 IU/1.

The present inventors discovered that a peptide consisting of the amino acid sequence represented by the formula pyroGlu-(X)n-A or a salt thereof (hereafter, such peptide is occasionally referred to as "the peptide of the present invention") has preventive and/or therapeutic activity for a liver disease. Herein, pyroGlu represents pyroglutamic acid; X may be the same or different and represent Gln (glutamine) or Asn (asparagine), and preferably Gln; A represents Gln, Asn, Leu (leucine), Ile (isoleucine), or Val (valine), and preferably Gln, Leu, or Ile; and n is 0, 1, or 2, and preferably 0 or 1. Examples of peptides represented by the above formula include pyroGlu-Gln, pyroGlu-Gln-Gln, pyroGlu-Leu, pyroGlu-Ile, pyroGlu-Gln-Leu, pyroGlu-Gln-Ile, pyroGlu-Gln-Gln-Gln, pyroGlu-Gln-Gln-Leu, and pyroGlu-Gln-Gln-Ile.

Pyroglutamic acid results from ring-closure of an amide group at the γ-position and an amino group at the α-position of glutamic acid. The peptide of the present invention may be a partial hydrolysate of a naturally-occurring or recombinant protein, a peptide prepared via chemical synthesis or genetic engineering, or a combination thereof.

As an amino acid that constitutes the peptide of the present invention, a D-form, L-form, or DL-form (i.e., a racemic form) amino acid can be used, and an L-form amino acid is particularly preferable. When the peptide of the present invention is prepared via partial hydrolysis of a naturally-occurring protein, all constituent amino acids are of L-forms. When the peptide of the present invention is prepared via chemical synthesis, a peptide consisting entirely of either L-amino acids or D-amino acids or a peptide comprising L-amino acids with the balance composed of D-amino acids can be prepared, and both types of peptides are within the scope of the peptide of the present invention.

The composition of the peptide of the present invention can be confirmed via amino acid analysis. In such a case, both pyroglutamic acid and glutamine are regarded as glutamic acids confirmed via a general acid hydrolysis method. Thus, it is preferable that glutamine and pyroglutamic acid be separately degraded with the use of a specific enzyme and then be quantified. When a peptide is a synthetic product, the composition can be determined based on the amount or percentage of amino acids used at the time of synthesis.

A salt of the peptide of the present invention is not particularly limited, provided that it is acceptable as a pharmaceutical or food product. Examples thereof include acid addition salts and base addition salts. Examples of acid addition salts include: salts with inorganic acids, such as hydrochloric acid, sulfuric acid, nitric acid, and phosphoric acid; and salts with organic acids, such as acetic acid, malic acid, succinic acid, tartaric acid, and citric acid. Examples of base addition salts include: salts with alkaline metals, such as sodium and potassium; salts with alkali earth metals, such as calcium and magnesium; and salts with amines, such as ammonium and triethylamine.

When the peptide of the present invention is prepared via partial hydrolysis of a naturally-occurring protein, a conventional protein hydrolysis method can be adequately performed. Specific examples include a method of hydrolysis using an acid and a method of hydrolysis using a protease.

Any available naturally-occurring proteins can be used for hydrolysis, and use of a protein with confirmed safety is preferable. Examples of such proteins include animal proteins derived from animal flesh, skin, milk, and blood, and plant proteins derived from grain crops, such as rice and wheat, and fruit, such as Japanese persimmon and peach. Among them, proteins such as gluten contained in wheat seeds are known to contain abundant glutamine, and such proteins are preferable as starting materials for preparing the peptide of the present invention.

A conventional method of protein hydrolysis involving the use of an acid can be employed. Examples of acids that can be used include: mineral acids, such as sulfuric acid, hydrochloric acid, nitric acid, phosphoric acid, and sulfurous acid; and organic acids, such as oxalic acid, citric acid, acetic acid, and formic acid.

When a protein is hydrolyzed using an acid, the protein concentration in an aqueous medium needs to be adequately adjusted in accordance with the acid type or normality. In general, the protein concentration is preferably adjusted to 1.0% to 80% by mass.

When a protein is hydrolyzed using a protease, one or more types of proteases may be allowed to act on the protein in an aqueous medium in order to generate a hydrolysate. A method involving the sole use of an acidic protease and a method involving the use of an acidic protease and a neutral protease or alkaline protease are preferable from the viewpoint of efficient hydrolysis. When a plant protein is used, starch or fiber content from plants may occasionally disturb protease action or purification. In such a case, it is preferable that a glycolytic enzyme such as amylase or cellulase be allowed to act on the protein before and after the treatment with protease, or together with a protease.

The thus-obtained protein hydrolysate can be purified via, for example, a method for filtering insoluble matter and a method of purification comprising fractionation (extraction) with the use of hydrous alcohol, followed by gel filtration chromatography or high-performance liquid chromatography (HPLC).

When the peptide of the present invention is prepared via chemical synthesis, either liquid-phase synthesis or solid-phase synthesis may be employed. Preferably, solid-phase synthesis is performed in which the C-terminus of an amino acid or peptide is immobilized via a linker to a solid-phase support and an amino acid is successively extended toward the N-terminus. When solid-phase synthesis is performed, a peptide synthesizer can be used (e.g., PSSM-8, Shimadzu and Model 433A, ABI).

Any solid-phase support can be used for solid-phase synthesis, provided that such support is capable of binding to a carboxyl group of the C-terminal amino acid of the peptide of the present invention; i.e., Gln, Aln, Leu, Ile, or Val. Examples include benzhydrylamine resin (BHA resin), chloromethyl resin, oxymethyl resin, aminomethyl resin, methylbenzhydryl resin (MBHA resin), acetamidomethyl resin (PAM resin), p-alkoxybenzyl alcohol resin (Wang resin), 4-aminomethyl-phenoxymethyl resin, and 4-hydroxylmethyl phenoxymethyl resin.

As a specific example of a synthesis technique, a procedure for preparing the peptide of the present invention; i.e., pyroGlu-Gln-Gln, is described below.

A C-terminal amino acid glutamine (Gln), with the carboxyl group is protected, and the second amino acid (Gln), with the amino group is protected by a protecting group such as a tert-butyloxycarbonyl (Boc) group or 9-fluorenyl-methoxycarbonyl (Fmoc) group, and the carboxy group is activated, are allowed to condensate. Subsequently, a protecting group of the amino group of the N-terminal glutamin of the generated Gln-Gln dipeptide is removed, then the third amino acid (Gln), with the amino group is protected by a protecting group such as a tert-butyl-oxycarbonyl (Boc) group or 9-fluorenyl-methoxycarbonyl (Fmoc) group, and the carboxy group is activated, is allowed to condensate. When solid-phase synthesis is employed, a carboxyl group of the glutamine as the C-terminal amino acid may be bound to a solid-phase support instead of protecting the carboxyl group.

A carboxyl group can be activated by allowing the carboxyl group to react with various types of reagents and generating corresponding acid chloride, acid anhydrate, or mixed acid anhydride, azide, or active esters of -ONp or -OBt. Also, the abovementioned peptide condensation can be carried out in the presence of a condensation agent or racemization inhibitor. Examples include a carbodiimide reagent such as dicyclohexylcarbodiimide (DCC), water-soluble carbodiimide (WSCD), and carbodiimidazole, tetraethyl pyrophosphate, and 1-hydroxybenzotriazole (HOBt).

After the completion of solid-phase synthesis, peptides are released from the solid-phase support, and all protecting groups are removed, followed by washing. Thus, a Gln-Gln-Gln tripeptide can be obtained in the form of a crude peptide. Subsequently, glutamine at the N-terminus can be converted into pyroglutamic acid via cyclization to obtain the peptide of the present invention. Cyclization gradually proceeds in an aqueous solution, and the speed of cyclization can be accelerated by raising the temperature. Also, peptides can be prepared by subjecting pyroglutamic acid as the N-terminal amino acid to condensation.

When liquid-phase synthesis is performed, peptides can be synthesized in the same manner as in the case of solid-phase synthesis, with the exception that the C-terminal amino acid is not bound to the solid-phase support. The thus-obtained crude peptide containing the peptide of the present invention can be adequately purified via a conventional technique, such as high-performance liquid chromatography (HPLC), to obtain a highly purified peptide.

As described above, the peptide of the present invention having the target amino acid sequence can be synthesized by successively subjecting amino acids to condensation and extension from the C-terminus toward the N-terminus via chemical synthesis of peptides. In this case, use of L- or D-forms of amino acids enables synthesis of a peptide comprising L-amino acids with the balance composed of D-amino acids.

The thus-obtained peptide of the present invention has high preventive and/or therapeutic activity for liver diseases. Thus, such peptide can be used as an active ingredient of the preventive and/or therapeutic composition for a liver disease. An embodiment of the present invention, accordingly, relates to a preventive and/or therapeutic composition for a liver disease comprising, as an active ingredient, the peptide of the present invention described above (hereafter, it may be referred to as "the composition of the present invention"). The composition of the present invention may comprise only one type or a plurality of types of the peptides of the present invention. Another embodiment of the present invention relates to a method for preventing and/or treating a liver disease comprising administering the peptide or composition of the present invention to a mammalian animal.

In the present invention, disease prevention includes suppression and delaying of disease development. The term "disease prevention" also includes prevention of recurrence of a disease after treatment, as well as prevention before disease development. In the present invention, treatment of a disease includes treatment of a disease, improvement of a symptom, and suppression of progression of a symptom.

In the present invention, the term "mammalian animals" refers to homeotherms. Examples thereof include primates such as humans and monkeys, rodents such as mice, rats, and rabbits, pet animals such as dogs and cats, and livestock animals such as cattle, horses, and pigs. The composition of the present invention is preferably administered to primates, and particularly preferably to humans. Particularly preferably, the composition of the present invention is administered to a human who is afflicted with a liver disease, a human who is diagnosed as having a liver disease, a human who may be afflicted with a liver disease, a human who is in need of prevention of a liver disease, and a human who has abnormal GOT and/or GPT levels (e.g., a human who has GOT and/or GPT levels that are higher than a normal level).

The composition of the present invention is generally administered in amounts of 0.01 g to 20 g and preferably 0.1 g to 10 g of peptides by mass to an adult per day. When the peptide used in the present invention is prepared via partial hydrolysis of a naturally-occurring protein, the dosage thereof can further be increased because of its high safety due to being derived from a naturally occurring substance. It is preferable that the dosage be adequately increased or decreased in accordance with the effects and other conditions. The dosage per day can be preferably administered or ingested at once or in several separated doses.

The form of the preventive and/or therapeutic composition for a liver disease of the present invention is not particularly limited. For example, such composition can be prepared in the form of a pharmaceutical composition or food product (including animal feeds).

When the composition of the present invention is prepared in the form of a pharmaceutical composition, in general, the composition is prepared as a pharmaceutical preparation comprising the peptide of the present invention and a pharmaceutically acceptable carrier. In general, the term "pharmaceutically acceptable carrier" refers to an inactive, atoxic, and solid or liquid filler, diluent, or encapsulating agent that does not react with the peptide of the present invention as an active ingredient. Examples thereof include water, ethanol, polyol (e.g., glycerol, propylene glycol, or liquid polyethylene glycol), a mixture of any thereof, and a solvent or dispersion medium such as a vegetable oil.

The dosage form of the pharmaceutical composition is not particularly limited, and an arbitrary dosage form can be employed. Examples thereof include dosage forms for oral administration, such as tablets, pills, granules, dust formulations, fine grains, powdered drugs, capsules, syrup preparations, drink preparations, liquid preparations, suppositories, and liquid meals, as well as dosage forms for parenteral administration, such as sublingual tablets, nasal sprays, and injection preparations.

The pharmaceutical composition of the present invention can be administered via administration techniques that are generally employed for pharmaceutical administration, such as intravenous administration, intramuscular administration, and subcutaneous administration, in addition to oral administration. Also, an administration technique that involves absorption through a mucous membrane other than the gastrointestinal tract, such as rectal, sublingual, or intranasal administration, can be employed. In such a case, the pharmaceutical composition can be administered in the form of, for example, a suppository, sublingual tablet, or nasal spray.

The content of the peptide of the present invention in the pharmaceutical composition varies depending on the form thereof. It is generally 0.001% to 99% by mass, preferably 0.01% to 90% by mass, more preferably 1% to 85% by mass, and further preferably 5% to 80% by mass, on a dry basis. It is preferable that the dosage per day be regulated, so as to realize the above-described dose for an adult per day.

When the composition of the present invention is prepared in the form of a food product, the form thereof is not particularly limited. The term "food product" refers to a beverage, a health food product, and a functional food product. Specifically, the health food product and the functional food product can be prepared in the form of various pharmaceutical preparations, such as tablets, pills, granules, dust formulations, fine grains, powdered drugs, capsules, syrup preparations, drink preparations, liquid preparations, and liquid meals. The food product in the form of a pharmaceutical preparation can be prepared in the same manner as in the case of the pharmaceutical composition. For example, an adequate excipient (e.g., starch, processed starch, lactose, glucose, or water) is added and the food product can then be produced via a conventional technique. Specific examples of food products further include coffee beverages, tea drinks, beverages containing fruit juice, soft drinks, milk beverages, butter, mayonnaise, shortening, margarine, various types of salad dressings, bread, noodles, cooked rice, pasta, sauce products, confectioneries, cookies, chocolates, candies, chewing gums, various types of seasonings, and various types of diet products. By incorporating the peptide of the present invention into such food product, the composition of the present invention may be prepared in the form of a food product.

The content of the peptide of the present invention in the food product of the present invention varies depending on the form of the food product, and it is generally 0.01 % to 80% by mass, preferably 0.1 % to 75% by mass, more preferably 1% to 70% by mass, and further preferably 5% to 70% by mass, on a dry basis. Since the peptide of the present invention is highly safe, the content thereof can further be increased. The amount thereof to be ingested per day may be administered at once or in several separated doses. It is preferable that the dosage per day be regulated, so as to realize the above-described dose for an adult per day.

Ingestion of the peptide of the present invention having preventive and/or therapeutic activity for a liver disease, a salt thereof, or the composition of the present invention comprising the same can produce the effects of blocking the advancement of liver disease conditions, the effects of ameliorating disease conditions, or the effects of preventing affliction with a liver disease.

The composition of the present invention can contain various additives that are used for production of pharmaceutical, food, and feed products, and various active substances can further be contained. Examples of such additives and active substances include various oils and fats, crude drugs, amino acids, polyhydric alcohol, naturally-occurring polymers, vitamins, minerals, dietary fibers, surfactants, purified water, excipients, stabilizers, pH modifiers, antioxidants, sweeteners, taste components, acidulants, colorants, and aroma chemicals. The peptide of the present invention can be administered in the form of a mixture thereof with one or more types of other active ingredients having preventive and/or therapeutic activity for a liver disease or in combination therewith. The preventive and/or therapeutic composition for a liver disease of the present invention, accordingly, may comprise other active ingredients having preventive and/or therapeutic activity for a liver disease, in addition to the peptide of the present invention.

Examples of the various types of oils and fats include vegetable oils and fats, such as soybean oil, safflower oil, and olive oil, and animal oils and fats, such as beef tallow and sardine oil.

Examples of the crude drugs include oriental bezoar, rehmanniae radix, lycii fructus, royal jelly, gensing, and Lurong.

Examples of the amino acids include cysteine, leucine, and arginine.

Examples of the polyhydric alcohol include ethylene glycol, polyethylene glycol, propylene glycol, glycerine, and sugar alcohol. Examples of sugar alcohol include sorbitol, erythritol, xylitol, maltitol, and mannitol.

Examples of the naturally-occurring polymers include gum Arabic, agar, water-soluble corn fiber, gelatin, xanthan gum, casein, gluten or gluten hydrolysate, lecithin, and dextrin.

Examples of various vitamins include vitamins A, D, and K and riboflavin tetrabutyrate, in addition to vitamin C (ascorbic acid), vitamin B family, and vitamin E (tocopherol). The vitamin B family includes various vitamin B complexes, such as vitamin B1, vitamin B1 derivative, vitamin B2, vitamin B6, vitamin B12, biotin, pantothenic acid, nicotinic acid, and folic acid. Vitamin B1 and derivatives thereof include all compounds having physiological activity of vitamin B1, such as thiamine or a salt thereof, thiamine disulfide, fursultiamine or a salt thereof, dicethiamine, bisbutytiamine, bisbentiamine, benfotiamine, thiamine monophosphate disulfide, cycotiamine, octotiamine, and prosultiamine.

Examples of the minerals include calcium, magnesium, zinc, and iron.

Examples of the dietary fibers include gums, mannan, pectin, hemicellulose, lignin, β-glucan, xylan, and arabinoxylan.

Examples of the surfactants include glycerine fatty acid ester, sorbitan fatty acid ester, and sucrose fatty acid ester.

Examples of the excipients include saccharose, glucose, corn starch, calcium phosphate, lactose, dextrin, starch, crystalline cellulose, and cyclodextrin.

Examples of other active ingredients having preventive and/or therapeutic activity for a liver disease include isoflavone, hyaluronic acid, valine, glutamine, folic acid, *Corbicula,* apple, loaches, and pears, in addition to the aforementioned pharmaceutical products.

In addition to the abovementioned, for example, taurine, glutathione, carnitine, creatine, coenzyme Q, α-lipoic acid, glucuronic acid, glucuronolactone, theanine, γ-aminobutyric acid, capsaicin, various organic acids, flavonoids, polyphenols, catechins, xanthine derivative, nondigestible oligosaccharides such as fructo-oligosaccharide, or polyvinylpyrrolidone may be added as additives. The amounts of such additives to be added are adequately determined in accordance with the additive type and the desirable amount to be ingested. In general, such amount is between 0.01 % and 30% by mass, and preferably 0.1 % to 10% by mass.

Production examples and test examples of the peptide and the composition of the present invention are described in detail below, although the present invention is not limited thereto.

### Examples

### (Production Example 1) Synthesis of pyroGlu-Gln-Gln

pyroGlu-Gln-Gln was synthesized by a solid-phase method using a Model 433A peptide synthesizer (ABI).

Automatic synthesis was carried out in the following manner using 2 g of Boc-Gln-Pam resin as a starting material and protected amino acids of Boc-Gln and Boc-Glu (OBzI).
(1) Removal of Boc group from Boc-Gln-Pam resin
(2) Washing
(3) Activation of Boc-Gln
(4) Condensation with the addition of activated Boc-Gln to GIn-Pam resin
(5) Washing
(6) Acetylation of unreacted N-terminal amino group
(7) Washing
(8) Removal of Boc group from Boc-Gln-Gln-Pam resin
(9) Washing
(10) Activation of Boc-Glu (OBzI)
(11) Condensation with the addition of activated Boc-Glu (OBzl) to Gln-Gln-Pam resin
(12) Washing
(13) Acetylation of unreacted N-terminal amino group
(14) Washing
(15) Boc-Glu(OBzl)-Gln-Gln-Pam resin

The Boc group was removed via treatment with trifluoroacetic acid-dichloromethane (50:50) for 20 minutes. The step of washing was repeated three times using dichloromethane. Condensation was carried out by adding the Boc-protected amino acids in amounts that were 5 times the equivalent of the resin-bound amino group and allowing the reaction to proceed for 60 minutes in the presence of DCC and HOBt.

The resulting Boc-Glu(OBzl)-Gln-Gln-Pam resin was removed from the peptide synthesizer and transferred to another vessel. Thioanisole (1 ml) and 0.5 ml of ethanedithiol were added per g of the resin, and the resultant was agitated at room temperature for 10 minutes. Subsequently, 10 ml of hydrogen fluoride was slowly added under ice cooling, the mixture was agitated for 30 minutes, and hydrogen fluoride was removed by distillation under reduced pressure. The reaction vessel was filled with 100 ml of cold diethylether, and the resultant was agitated for 1 minute to precipitate peptide and resin. The resultant was collected via filtration with a Polyfron filter PF060 (Advantec Co., Ltd.) and washed with cold diethylether (-40°C). Peptide was dissolved in about 30 ml of trifluoroacetic acid; and the resulting peptide solution was added dropwise to 300 ml of cold diethylether, which had been prepared in advance, followed by reprecipitation. The resultant was collected via filtration with a PTFE membrane (pore size: 3 µm, Advantec Co., Ltd.), the resultant was washed with cold diethylether (-40°C), and peptide was dissolved in 2N acetic acid, followed by lyophilization. Crude peptide (1.21 g) was obtained from 2.35 g of the protected peptide-Pam-resin. Crude peptide was dissolved in water, and the resultant was subjected to cyclization to pyroglutamic acid at 60°C for 6 hours, followed by lyophilization.

The resulting crude peptide was purified via HPLC under the following conditions.

Column: Inertsil ODS-3, ϕ20 × 250 mm (GL Sciences Inc.)

Mobile phase: Gradient from 0.1 % trifluoroacetic acid to 35% acetonitrile in 0.1 % trifluoroacetic acid

Flow rate: 10 ml/min

Detector: Ultraviolet spectrophotometer, 210 nm

Temperature: 40°C

The main peak of the HPLC chromatogram was fractionated, and the amino acid sequence of the fractionation product was analyzed using a peptide sequencer. From 1 g of crude peptide, 0.88 g of purified peptide of pyroGlu-Gln-Gln was obtained. (Production Example 2) Synthesis of pyroGlu-Leu pyroGlu-Leu was synthesized by the Boc liquid-phase method.

### (1) Condensation of Boc-pyroGlu and HCl Leu-O^{t}Bu

HCl Leu-O^{t}Bu (390 mg) was introduced into an eggplant-shaped flask, dissolved in 5 ml of DMF, and ice-cooled, following which 0.124 ml of triethylamine was added. Subsequently, 400 mg of Boc-pyroGlu-OH, 470 mg of HOBt, and 367 mg of WSCD HCl were added, and the mixture was agitated for 12 hours under ice cooling to perform a condensation reaction. After the completion of the reaction, DMF was removed by distillation under reduced pressure, the residue was dissolved in ethyl acetate, ethyl acetate was successively washed with 5% sodium hydrogen carbonate aqueous solution, 10% citric acid aqueous solution, water, and saturated saline, and the resultant was dried over anhydrous sodium sulfate. Sodium sulfate was separated via filtration, the filtrate was concentrated under reduced pressure, and ether-hexane was added to the resulting residue to solidify and collect Boc-pyroGlu-Leu-O^{t}Bu. The yield was 609 mg (88%).

### (2) Deprotection

The Boc-pyroGlu-Leu-O^{t}Bu (600 mg) obtained above was introduced into an eggplant-shaped flask, 5 ml of trifluoroacetic acid was added to dissolve the Boc-pyroGlu-Leu-O^{t}Bu, and deprotection was carried out for 1 hour under ice cooling. Trifluoroacetic acid was removed with the use of N₂ gas, and the deprotected peptide was solidified with the addition of ether, followed by collection via filtration. The resulting solid was dissolved in 4N HCl/dioxane, and ether was added for resolidification, followed by collection via filtration. The yield was 220 mg (53%).

### (Production Example 3) Synthesis of pyroGlu-Ile

pyroGlu-Ile was synthesized in the same manner as in Production Example 2 with the use of 390 mg of HCl H-Ile-O^{t}Bu as a starting material. The yield of the condensation reaction was 613 mg (88%), and the yield of the deprotected peptide was 260 mg (67%).

### (Production Example 4) Synthesis of pyroGlu-Gln-Gln

pyroGlu-Gln-Gln was synthesized by the Fmoc liquid-phase method.

### (1) Synthesis of Fmoc-Gln(Trt)-Gln-O^{t}Bu

HCl H-Gln-O^{t}Bu (1.15 g) was introduced into an eggplant-shaped flask, dissolved in 5 ml of DMF, and ice cooled, following which 0.74 ml of triethylamine was added. Subsequently, 2.94 g of Fmoc-Gln(Trt)-OH, 1.3 g of HOBt, and 1.01 g of WSCD HCl were added, and the mixture was agitated for 12 hours under ice cooling to perform a condensation reaction. After the completion of the reaction, DMF was removed by distillation under reduced pressure, the residue was dissolved in ethyl acetate, ethyl acetate was successively washed with 5% sodium hydrogen carbonate aqueous solution, 10% citric acid aqueous solution, water, and saturated saline, and the resultant was dried over anhydrous sodium sulfate. Sodium sulfate was separated via filtration, the filtrate was concentrated under reduced pressure, and ether-hexane was added to the resulting residue to solidify and collect Fmoc-Gln(Trt)-Gln-O^{t}Bu. The yield was 3.51 g (92%).

### (2) Removal of Fmoc group from Fmoc-Gln(Trt)-Gln-O^{t}Bu

Fmoc-Gln(Trt)-Gln-O^{t}Bu (1.12 g) was introduced into an eggplant-shaped flask, and 7 ml of 1M NaOH aqueous solution was added under ice cooling. Since the mixture developed a white turbidity, methanol was added to dissolve the contents thereof, and the reaction was allowed to proceed at 0°C for 2 hours. After neutralization with the addition of citric acid, water was added to a white solid resulting from vacuum concentration, and the mixture was agitated to obtain a gummy solid. The resultant was applied to a silica gel column using a chloroform solvent, and the target component was fractionated and solidified with the aid of ether. The yield was 590 mg (73%).

### (3) Synthesis of Boc-pyroGlu-Gln(Trt)-Gln-O^{t}Bu

H-Gln(Trt)-Gln-O^{t}Bu (580 mg) was introduced into an eggplant-shaped flask, dissolved in 5 ml of DMF, and ice cooled, following which 156 µl of triethylamine was added. Subsequently, 232 mg of Boc-pyroGlu-OH, 273 mg of HOBt, and 213 mg of WSCD HCl were added, and the mixture was agitated for 12 hours under ice cooling to perform a condensation reaction. DMF was removed by distillation under reduced pressure, the residue was dissolved in ethyl acetate, ethyl acetate was successively washed with 5% sodium hydrogen carbonate aqueous solution, 10% citric acid aqueous solution, water, and saturated saline, and the resultant was dried over anhydrous sodium sulfate. Sodium sulfate was separated via filtration, the filtrate was concentrated under reduced pressure, and the solvent was removed from the resulting residue by reducing a pressure using a vacuum pump. The yield was 509.3 mg (64%).

### (4) Deprotection

Boc-pyroGlu-Gln(Trt)-Gln-O^{t}Bu (760 mg) was introduced into an eggplant-shaped flask, 10 ml of trifluoroacetic acid was added to dissolve Boc-pyroGlu-Gln(Trt)-Gln-O^{t}Bu, and the reaction was allowed to proceed for 4 hours under ice cooling. Trifluoroacetic acid was removed with the use of N₂ gas, and the deprotected peptide was solidified with the addition of ether. The solid was recovered via centrifugation, ether was added again to suspend the solid, and the solid was sampled via centrifugation. This procedure was repeated three times to obtain crude peptide. The yield was 445 mg (100%).

### (5) Purification of pyroGlu-Gln-Gln

The crude peptide obtained above contained water-insoluble impurities. Thus, the crude peptide was suspended in water, and the filtrate was collected through a filter. To the filtrate, 2 ml of 1M hydrochloric acid was introduced and lyophilized. Ether was added to the lyophilization product to solidify the peptide of the present invention, and the solid was recovered and then dried. The final yield was 256 mg (63%).

### (Production Example 5) Extraction of pyroGlu-Gln-Gln, pyroGlu-Gln, pyroGlu-Leu, and pyroGlu-Ile from naturally-occurring protein

(1) Ion-exchanged water (9,700 kg), 38 kg of anhydrous citric acid, and 1,500 kg of wheat gluten (active gluten, Weston Foods Limited) were introduced into a reaction vessel, the temperature was raised to 45°C, 2.2 kg of protease (Protease M Amano, Amano Pharmaceutical Co., Ltd.) and 1.1 kg of amylase (a liquefying enzyme T, Hankyu Bioindustry Co., Ltd.) were added, and hydrolysis was carried out at 45°C for 5 hours. Subsequently, the liquid pH level was adjusted to 4.4 to 4.5 with the use of 25% sodium hydroxide aqueous solutions, the liquid was retained in such state for 7 hours, and an enzyme treatment was then carried out.
(2) Subsequently, the liquid was maintained at 80°C for 20 minutes to deactivate a protease, the liquid was cooled to 65°C, 0.5 kg of amylase (a liquefying enzyme T, Hankyu Bioindustry Co., Ltd.) was added thereto to hydrolyze starch and fiber components contained in the wheat gluten, and the liquid was maintained at 90°C for 20 minutes to deactivate amylase.
(3) Subsequently, the liquid was cooled to 10°C or lower, the liquid was heated to 55°C again, 100 kg of active carbon (Takecoal, Takeda Pharmaceutical Company Limited) was added thereto, and the mixture was agitated at 55°C for 30 minutes.
(4) The liquid temperature was adjusted to 45°C, a filter aid (Radiolite, Showa Chemical Industry Co., Ltd.) was added, and filtration was carried out using a pressure filtration apparatus to recover 7,000 liters of filtrate (7 m³).
(5) The filtrate recovered in (4) above was vacuum concentrated, the concentrate was sterilized by heating at 110°C for 20 seconds using a plate heater, and the resultant was then cooled to 55°C.
(6) The liquid obtained in (5) above was spray-dried using a spray drier at a blast temperature of 160°C and an air exhaust temperature of 80°C to obtain about 1,000 kg of powder of wheat gluten hydrolysates.
(7) Fractions with molecular weights of 1,000 or smaller were fractionated from the powder obtained in (6) above via gel filtration, and purification was further carried out via HPLC. Via HPLC, portions exhibiting the same retention time under the same conditions were recovered based on the synthesized pyroGlu-Gln-Gln, pyroGlu-Gln, pyroGlu-Leu, and pyroGlu-Ile obtained in the same manner as in Production Example 1. As a result, 4.5 kg, 1.6 kg, 0.9 kg, and 0.7 kg of peptides were obtained from 800 kg of powdered hydrolysates of wheat gluten.
(8) The amino acid sequences of the purified peptides were analyzed using a peptide sequencer. As a result, the peptides were found to contain sequences pyroGlu-Gln-Gln, pyroGlu-Gln, pyroGlu-Leu, and pyroGlu-Ile, respectively.

### (Example 1) Production of tablet

The pyroGlu-Gln peptide (84 g) obtained in Production Example 5, 10 g of crystalline cellulose (Asahi Kasei Corporation), and 5 g of polyvinylpyrrolidone (BASF) were mixed, 3 ml of ethanol was added thereto, and granules were produced by a conventional wet method. The resulting granules were dried, 1.1 g of magnesium stearate was added thereto to prepare dust for tablet production, tablets were prepared using a tableting machine, and 100 tablets each having a weight of 1 g were produced (pyroGlu-Gln content per tablet: 0.84 g).

### (Example 2) Production of syrup preparation

Purified water (400 g) was boiled, 750 g of saccharose and 100 g of the pyroGlu-Leu peptide obtained in Production Example 5 were added and dissolved therein during stirring, the solution was subjected to straining while being maintained in a heated state, purified water was added thereto to bring a total amount to 1,000 ml, and syrup preparations were produced (pyroGlu-Leu content per 100 ml of syrup preparation: 10 g).

### (Example 3) Production of granular preparations

PyroGlu-Ile peptide obtained in Production Example 5 (76 g), 13.3 g of lactose (DMV), 6.7 g of crystalline cellulose (Asahi Kasei Corporation), and 4 g of polyvinylpyrrolidone (BASF) were mixed, 30 ml of ethanol was added thereto, and granules were produced in accordance with a conventional wet method. After drying, granular sizes were adjusted to obtain granular preparations (pyroGlu-Ile content per 10 g of granular preparation: 7.6 g).

### (Example 4) Production of liquid meal

Sodium caseinate (DMV, 40 g), 160 g of maltodextrin (Sanwa Cornstarch Co., Ltd.), and 25 g of pyroGlu-Ile peptide obtained in Production Example 5 were added and dissolved in 750 ml of purified water at about 65°C. Subsequently, 5 g of vitamin mix and 5 g of mineral mixture comprising sodium, potassium, calcium, magnesium, chlorine, iron, phosphorus, copper, zinc, manganese, and sulfide were added. The mixture was introduced into a homo mixer (Tokushu Kika Kogyo Co., Ltd.) and roughly emulsified at about 8,000 rpm for 15 minutes. The resulting emulsion was cooled to about 20°C, aroma chemicals were added, and the resultant was diluted in a measuring cylinder to the final amount of 1,000 ml. A pouch was filled with the emulsion (230 g), the pouch was hermetically sealed while conducting nitrogen gas substitution, and the liquid was sterilized at 121°C for 15 minutes to obtain a concentrated liquid meal (pyroGlu-Ile content per 230 g of liquid meal: about 5.8 g).

### (Example 5) Production of bread

Wheat flour (bread flour) (150 g) was mixed with 2 g of dry yeast. In addition, 20 g of the pyroGlu-Gln-Gln peptide obtained in Production Example 5, 20 g of sugar, 3 g of salt, and 6 g of skimmed milk powder were dissolved in 70 g of warm water, chicken egg was added thereto, and the resultant was thoroughly mixed. The resultant was added to the wheat flour, the mixture was thoroughly kneaded by hand, about 40 g of butter was added thereto, the mixture was further kneaded, and dough for 20 roll buns was prepared. The dough was allowed to rise, a beaten egg was applied to the surface, and the resultant was baked in an oven at 180°C for about 15 minutes to prepare roll buns (pyroGlu-Gln-Gln content per roll bun: about 1 g).

### (Example 6) Production of meat sauce for pasta

One serving of a meat sauce for pasta (150 g) was introduced into a pan, 5 g of the pyroGlu-Gln-Gln peptide obtained in Production Example 5 was simultaneously added, and the resultant was heated to prepare a meat sauce for pasta. A pouch was filled with the resulting sauce, the pouch was hermetically sealed while conducting nitrogen gas substitution, and the sauce was sterilized at 121 °C for 15 minutes to obtain a meat sauce for pasta containing the pyroGlu-Gln-Gln peptide.

### (Example 7) Production of Japanese wheat noodle

The dispersion of 15 g of the pyroGlu-Gln peptide obtained in Production Example 5 and 15 g of salt in 150 g of water was added to 300 g of wheat flour (all-purpose flour), and the resultant was thoroughly kneaded and allowed to stand. Thereafter, the dough was stretched and sliced to a width of about 5 mm to prepare Japanese wheat noodles. The resultant was cooked in boiling water for about 10 minutes. The resultant Japanese wheat noodles exhibited good appearance, taste, and texture. The Japanese wheat noodles contained about 5 g of pyroGlu-Gln peptide per serving.

### (Test Example 1) Effects on liver disease (administration test using hepatic disorder rat model)

### (1) Test method

Wistar rats (4- to 5-week old) were used for the experiment. Rats were preliminarily fed for a week with purified standard feeds and subjected to fasting for 4 hours. pyroGlu-Leu obtained in Production Example 2 and pyroGlu-Ile obtained in Production Example 3 were each dissolved in water at 10 mg/l ml, and the resulting solutions were administered intraperitoneally in amounts of 20 mg/kg each. D-galactosamine (Sigma) was administered intraperitoneally in an amount of 800 mg/kg 1 hour later. Rats were allowed to freely eat 4 hours after D-galactosamine administration. Each group consisted of 5 rats, and water was administered to the control group instead of the peptide of the present invention.

Blood was sampled from the rats 24 hours after D-galactosamine administration, and GOT and GPT levels in the blood were assayed to compare the degrees of hepatic disorders.

GOT and GPT levels were assayed in accordance with the JSCC standardization method (Hiroshi Miura, Japanese Journal of Clinical Medicine (Nippon Rinsho), 53-reprinted-266, 1995). The results of analysis are shown in Table 1.

**Table 1**

| | GOT (IU/L) | GPT (IU/L) |
|---|---|---|
| Water | 391 | 50 |
| pyroGlu-Leu | 215 | 30 |
| pyroGlu-Ile | 314 | 45 |

The results shown in Table 1 demonstrate that the peptide of the present invention exhibits strong activity of suppressing increase in GOT and GPT and activity of suppressing hepatic disorders, i.e., liver diseases.

### (Test Example 2) Effects on liver disease (administration test using hepatic disorder rat model)

### (1) Test method

The test was carried out in the same manner as in Test Example 1. pyroGlu-Gln-Gln, pyroGlu-Gln, pyroGlu-Leu, or pyroGlu-Ile obtained in Production Example 5 was dissolved in water at 10 mg/l ml, and the resulting solution was administered orally to rats in amounts of 40 mg/kg. The results are shown in Table 2.

**Table 2**

| | GOT (IU/L) | GPT (IU/L) |
|---|---|---|
| Water | 2180 | 391 |
| pyroGlu-Gln-Gln | 524 | 100 |
| pyroGlu-Gln | 509 | 124 |
| pyroGlu-Leu | 555 | 131 |
| pyroGlu-Ile | 491 | 98 |

The results shown in Table 2 demonstrate that the peptide of the present invention exhibits strong activity of suppressing increases in GOT and GPT and activity of suppressing hepatic disorders, i.e., liver diseases.

All publications, patents, and patent applications cited herein are incorporated herein by reference in their entirety.

## Claims

1. A peptide consisting of the amino acid sequence represented by the following formula:
pyroGlu-(X)n-A
wherein X is the same or different and represents Gln or Asn; A represents Gln, Asn, Leu, Ile, or Val; and n is an integer from 0 to 2, or a salt thereof.

2. The peptide or salt thereof according to claim 1, wherein X represents Gln; A represents Gln, Leu, or Ile; and n is 0 or 1.

3. A preventive and/or therapeutic composition for a liver disease comprising, as an active ingredient, at least one of the peptides or salts thereof according to claim 1 or 2.

4. The composition according to claim 3, wherein the liver disease is viral hepatitis, an alcoholic liver disease, or a drug-induced liver disease.

5. The composition according to claim 3 or 4, which is in the form of a food product.
